# EUROPEAN PATENT SPECIFICATION

(11) **EP 4 430 986 B1**
(45) Date of publication and mention of the grant of the patent: **10.09.2025**
(21) Application number: 24162692.8
(22) Date of filing: 11.03.2024
(51) Int. Cl.: A45D 40/00, A61Q 1/02, A61K 8/02

(54) **MAKE-UP PRODUCT**
SCHMINKPRODUKT
PRODUIT DE MAQUILLAGE

(30) Priority: 14.03.2023 IT 202300004752
(43) Date of publication of application: 18.09.2024
(73) Proprietor: Chromavis S.p.A., 26010 Offanengo (CR) (IT)
(72) Inventor: Guizzetti, Giorgio, 20871 Vimercate (MB) (IT); Gaboardi, Mauro, 26026 Pizzighettone (CR) (IT); Raffaldi, Samuele, 26861 Fombio (LO) (IT); Valvassori, Sergio, 26010 Ripalta Cremasca (CR) (IT)
(74) Representative: Vanosi, Adelio Valeriano

(56) References cited:
- EP-A1- 3 909 561
- EP-A1- 4 062 794
- WO-A1-2022/195339
- JP-B2- 4 027 869
- US-A1- 2021 353 035

## Description

### FIELD OF THE INVENTION

The present invention relates to a make-up product (or for make-up).

In particular, the invention refers to a make-up product such as a pressed powder, a cast product (foundation, lipsticks, concealers metered into a godet or jar) or baked (wet powders whose production process involves a drying step in an oven) or a stick for lip use.

### STATE OF THE ART

Make-up products which have a three-dimensional surface are commonly known, for example those disclosed in EP4062794-A1 and EP3909561-A1.

The three-dimensional surface of these cosmetics allows two different patterns or words to be highlighted on the surface of the product, depending on the observation angle.

This feature makes the products more attractive visually, thanks to the dynamism of the images on the surface thereof.

However, make-up product customers are always looking out for new aesthetic effects that do not involve exclusively colour or simple surface decorations. A make-up product with a substantially planar surface defined by a plurality of three-dimensional elements is known from WO 2022/195339 A1.

### SUMMARY OF THE INVENTION

The object of the present invention is to provide a make-up product which is improved with respect to the prior art.

A further object of the invention is to provide a make-up product which appears different when observed from three different angles (or viewpoints).

This and other objects are achieved by means of a make-up product produced according to the technical teachings of the claims annexed hereto.

### BRIEF DESCRIPTION OF THE FIGURES

Further features and advantages of the innovation will become clearer in the following description of a preferred but not exclusive embodiment of the make-up product, illustrated - by way of a non-limiting example - in the drawings annexed hereto, in which:
Figure 1 is a plan view of the make-up product according to the present invention;
Figure 2 is a simplified section taken along the line II-II in Figure 1;
Figure 3 is a simplified section taken along line III-III in Figure 1;
Figure 4 is a schematic enlarged view of the detail enclosed in the circle in Figure 1;
Figure 5 is a perspective view of the make-up product in Figure 1 inserted in a cosmetic container;
Figure 6 is a perspective view, taken from a further angle, of the make-up product in Figure 5;
Figure 7 is a still further perspective view, taken from a still further angle, of the make-up product in Figure 1;
Figure 8 is a perspective view of a possible embodiment of the make-up product according to the present invention;
Figure 9 is a perspective view of a section of the product in Figure 8.

### DETAILED DESCRIPTION OF THE INVENTION

With reference to the figures stated, reference number 1 is used to denote, as a whole, a make-up product.

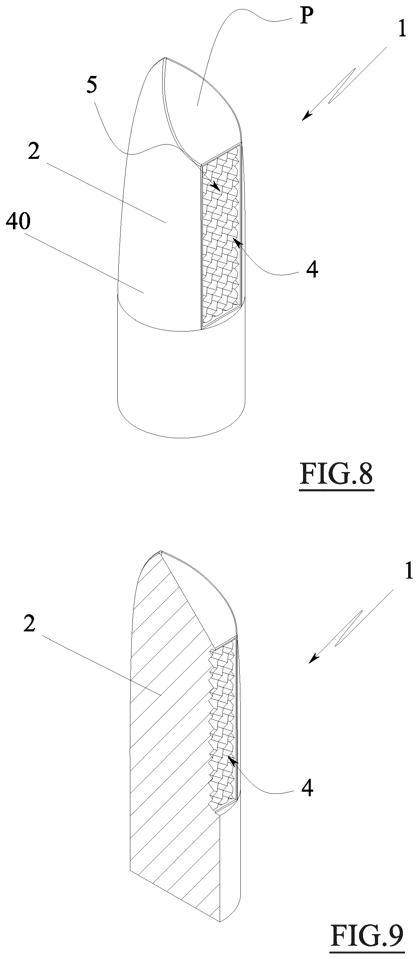

The make-up product 1 comprises a make-up substance 2 with its own shape (for example the stick in Figure 8) endowed with at least one substantially planar surface 4.

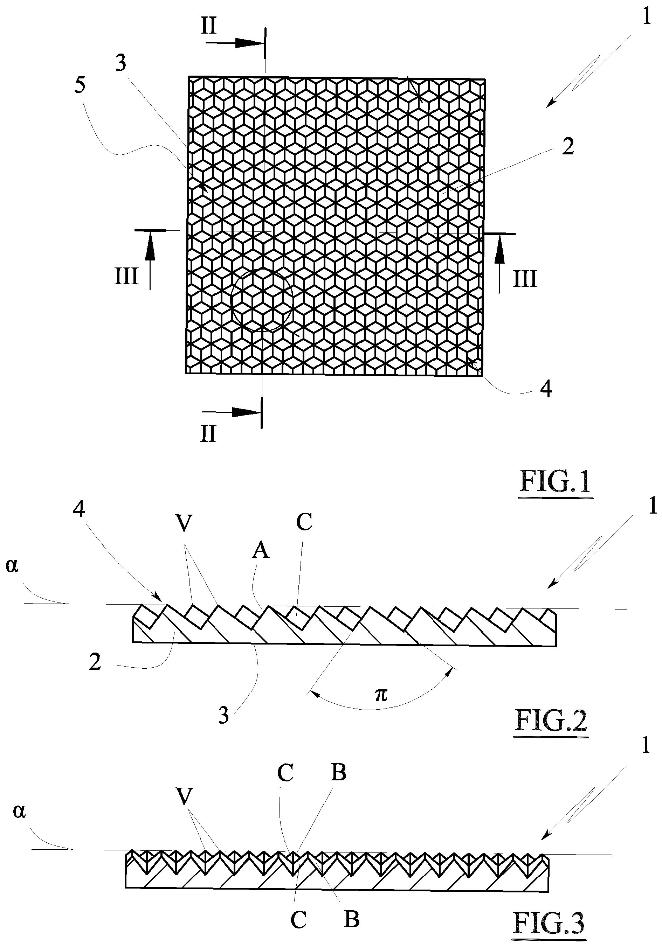

Or the make-up substance 2 is arranged, as in Figures 1-7, on a substantially flat support 3. Also in this case, the make-up substance has at least one substantially planar surface 4.

It should be noted that the substantially planar surface 4 (or briefly, surface 4) is formed of the said make-up substance 2 and therefore is also make-up, in the sense that if handled the said surface releases part of the constituent make-up substance.

The make-up substance 2 may be, for example, a compact cosmetic powder (or a mix of cosmetic powders) or a product cast into a godet, stick, etc.

Therefore the make-up product may be a blusher, a foundation, a powder, an eye shadow, a lipstick etc.

Some examples of the make-up substance 2 are listed below together with, consequently, some examples of the cosmetic products made therewith.

The make-up substance 2 may be a compact product.

This product comprises a powder part formed of one or more of the following elements: excipients, pigments, texturisers, silica, mica, beads, sericite, talc, and kaolin.

The product also includes a liquid/waxy part that functions as a binder. This part is present in a smaller amount, for example between 3% and 10% of the total product weight.

The powder is mixed with the binder, then sieved to improve the particle size and pressed into special primary packaging (mainly godets made of metal or of a further material which is resistant to deformation) in order to lend the end product a solid (compact) consistency.

In this case, the shape of the make-up substance 2 may be a pod (a disk or a three-dimensional parallelepiped whose height is minimal with respect to the width and length).

The make-up substance 2 may also be a cast product.

This product comprises a powder part which may include one or more of the following: pigments, texturisers, natural or synthetic micas, pearlescent powders, matting agents, and elastomeric resins.

The powder part is amalgamated with an anhydrous base, comprising for example one or more of the following: waxes, oils, esters, silicones, alkanes, polymer, and an aqueous base comprising one or more of the following: water, emulsifiers, waxes, oils, esters, silicones, etc.

The base, in this case, generally makes up 2/3 of the total product weight.

The mixture comprising the powder part and the anhydrous/aqueous part is in a liquid form when subjected to temperatures above the melting point of the waxes contained therein, but is substantially solid at room temperature (25°C). The overheated product (therefore, in liquid form) is cast into specific moulds made of metal or a further material, in which the said product solidifies after cooling (e.g. lipstick or sticks for lip use). The solidified product is then extracted from the moulds.

In this case, the make-up substance can take the form of a stick: this is a product which is cast in the form of a stick and usually positioned in tubular containers (known as devices) equipped with mechanisms that allow vertical movement of the product so that the product can be used (in the extracted position) and then replaced in the container (in the retracted position). In this case, the stick must have at least one substantially planar surface 4.

The heated liquid product can also be poured into moulds with another form, in addition to that provided for lipsticks and sticks for lip use, for example a prismatic form (cylinder, cube, parallelepiped, etc.). Also in this case, the make-up substance 2 has at least one planar surface 4 which is exposed and therefore accessible from the primary container (for example a godet or jar).

Again, the make-up substance 2 may be a baked product.

This product comprises a powder part formed of one or more of the following: excipients, pigments, texturisers, silica, mica, beads, sericite, talc, kaolin, and a binding part which may contain, for example, water, silicones, emulsifiers, waxy substances, or a combination thereof.

The mixture obtained from mixing the powder part and the binding part is elastic and pasty and can be metered onto a special plate or into a further container.

Once metered out, the said mixture can be pressed to obtain a desired relief or design and then left to dry until the solvent component (water or other volatile silicones) has evaporated almost completely.

After drying, the solvent component may amount to between 1% and 0.2%, generally 0.8%.

In this case, the shape of the make-up substance 2 may be a pod (a disk or a three-dimensional parallelepiped whose height is minimal with respect to the width and length).

The make-up substance 2 may also be an extruded product.

This is a product comprising a powder part constituted of pigments, excipients, mica, silica, sericite, texturisers, and a fluid binding part which may contain, for example, silicones, esters, water, emulsifiers, or waxy substances. Mixing these ingredients gives rise to an elastic paste which can be extruded into the tab shape, die-cut, and placed in a specific container (metal godet or jar). The product may be undergo pressing to give the surface a particular pattern, relief, or design.

The product is then left to dry until the volatile component of the solvent has almost completely evaporated (residue between 1% and 0.2%, generally 0.4%).

In this case, the shape of the make-up substance 2 may be a pod, or a three-dimensional parallelepiped whose height is comparable to the width and/or length (a chalk), or a small cylinder.

In the case of a small cylinder, at least one part must be substantially planar.

Initially, reference is made to Figures 1-7, which could show a compact product, a baked product, or an extruded product.

The make-up substance 2 features at least one make-up application surface, which may be the largest freely accessible surface, from which the user takes the said substance (using their hand or an applicator) to apply the make-up.

At least part of the make-up application surface of the make-up product is formed of a substantially planar (or flat) surface 4.

The substantially planar surface 4 is formed of a plurality of three-dimensional elements 5, 5', 5" arranged side by side.

In practice, the three-dimensional elements can be obtained on the substantially planar surface 4 which is visible.

It should be noted that the presence of the three-dimensional elements 5 on the substantially planar surface 4 does not affect the fact that the said surface can be deemed "planar" or "flat". Indeed, the height of the three-dimensional elements is negligible, determining simply a coarseness of the visible surface.

The three-dimensional elements 5, 5', 5'' may be arranged over the entire make-up application surface, if the said surface is substantially planar, or over solely part thereof.

According to the invention, each three-dimensional element 5 is defined by a first face A, a second face B, and a third face C.

The three faces A, B, C are regular squares arranged like three faces of a regular cube with a common vertex V.

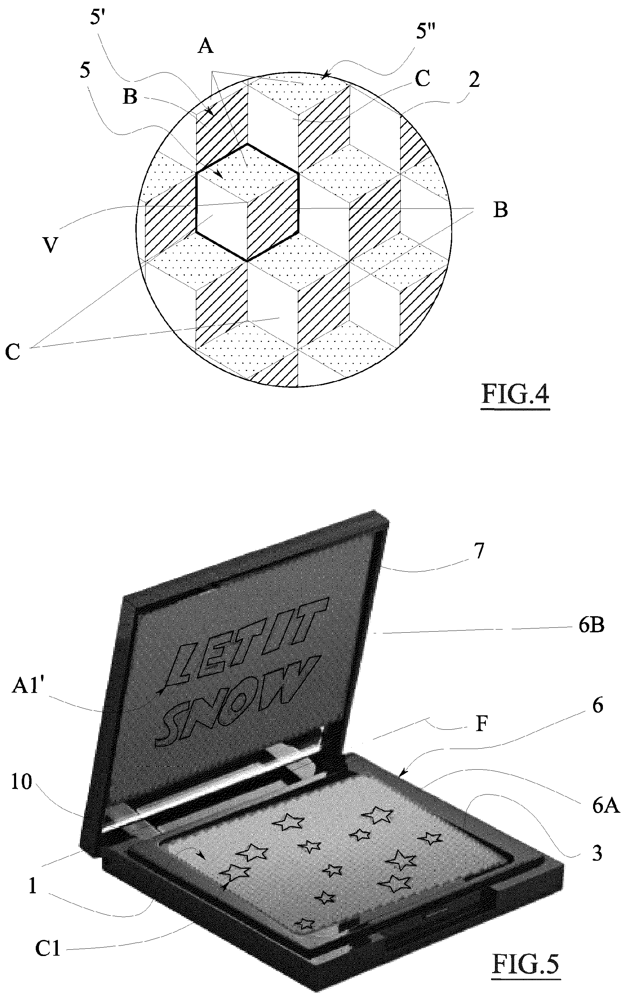

Therefore, as can be seen in Figure 4, in relation to element 5, face A has one edge in common with face B of the same element and one edge in common with face C of the same element. Faces B and C of the same element 5 share one mutual edge.

Face A of each element, meanwhile, has one edge in common with face B of a neighbouring element 5' and another edge in common with face C of another neighbouring element 5'', and so on for all the three-dimensional elements of the surface 4, as is clearly shown in Figure 4.

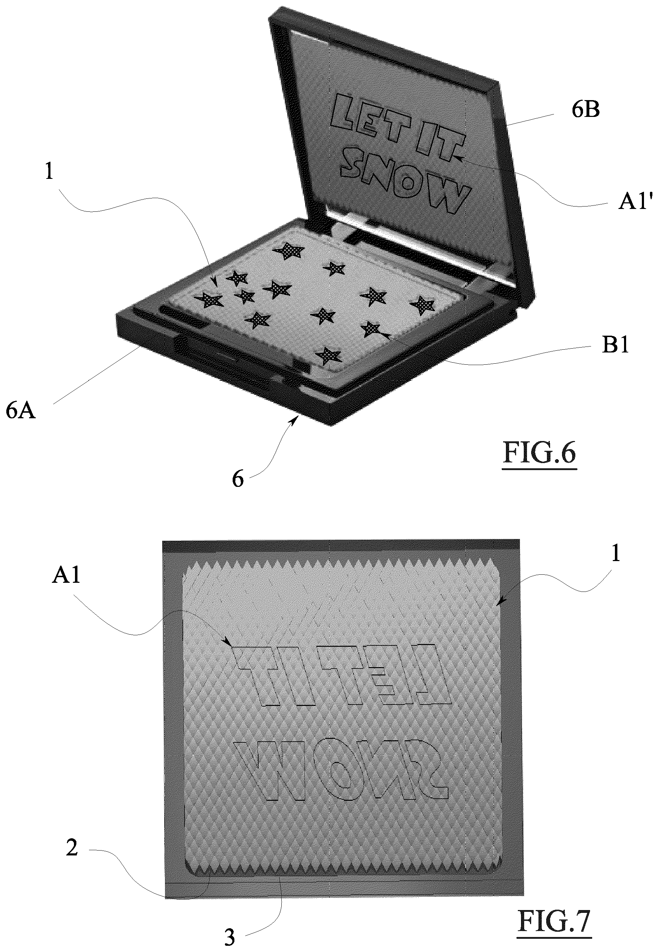

A first decoration A1 is created on the set of first faces A. In the example, the words 'LET IT SNOW' are visible as if 'reflected' when a user views the surface 4 from a substantially normal direction with respect to the first faces A (Fig. 7, or un-reflected -right- when the image is viewed in the mirror as in Figures 5 or 6).

A second decoration B1 is created on the set of second faces B (in the example, solid stars) which is visible when a user observes the surface 4 from a substantially normal direction, respect to the second faces B, as in Figure 6.

A third decoration C1 is created on the set of third faces C (in the example, empty stars) which is visible when a user observes the surface 4 from a substantially normal direction with respect to the third faces C (Figure 5).

Obviously, the decorations A1, B1, and C1 shown here are merely examples.

Advantageously, the vertices V of all the three-dimensional elements lie on a single plane α, as can be seen in Figures 2 and 3**.**

From Figure 5, it can be seen that the support 3 can be a flat surface housed or delimited in a base element 6A of a container 6 (for cosmetics) equipped with a lid 6B hinged to the base element 6A along an axis F**.**

To make the effect of Figures 5 and 6 possible, the lid 6B can hold a mirror 7 which is facing - at least when the container is open - the said surface 4.

The lid 6B can, advantageously, have an opening limiter 10, which stops the said lid opening at a certain angle with respect to the surface 4, more specifically when the mirror is angled so that a normal thereof is parallel to the normal of said first faces A**.**

It should be noted that, in the event of a configuration of this kind, the first decoration A1 is made so as to be legible when reflected in the mirror 7.

In the drawings annexed hereto, the decoration(s) A1, B1 and C1 are words (A1) and designs (B1, C1). However, the decorations may be of any kind or shape, including letters, numbers, designs, shading, colour effects, logos, etc.

The three-dimensional elements 5**,** 5', 5'' can be created on the surface in various ways.

For example, if the make-up substance is a compact powder, at least the last pressing step or steps can be carried out using a mould which reproduces, in three-dimensional negative form, the surface to be imprinted on the cosmetic powder.

The mould, at least the surface that imprints the shape (in negative) thereof onto the powder, can be made with the following technologies, either individually or combined:
1- Mechanical chip removal processing
2- Acid treatment
3- Surface treatment
4- Laser processing
5- Pressing

It has been shown that an invention such as the one described provides a make-up substance endowed with a surface 4 which has three different appearances when observed from three different directions.

Reference will now be made to Figures 8 and 9, which illustrate a stick for lip use, therefore a cast make-up product.

In this case, as already mentioned, the said product has at least one substantially planar surface 4 (used to perform a make-up operation) on which the said three-dimensional elements 5 described in the previous embodiment are made.

As can be seen from the drawings, only part of the entire make-up application surface of the stick has a planar configuration, featuring the three-dimensional elements 5. The rest of the surface 40 of the stick (also for applying the make-up) can have a more conventional shape, for example rounded or convex, as usual in this type of product.

Therefore, also in the stick shown in Figures 8 and 9, due to the three-dimensional elements 5, the surface 4 has the same (or a similar) visual effect as described for the first embodiment.

For example, the three-dimensional elements can be positioned so that a first design is visible when observing the surface 4 from the tip of the stick, a second is visible when viewing the surface from the right-hand side, and a third is visible when viewing from the left-hand side.

It should be noted that the surface 4 (endowed with three-dimensional elements 5) was created on the sides of the stick, but there is nothing to prevent the three-dimensional elements also (or only) being envisaged at the tip P of the stick, exploiting the planarity characteristics thereof.

To make the stick described above, it is possible to create a male which, in turn, has a planar surface (which will feature the three-dimensional elements 5 in negative) using the following technologies, either individually or combined:
1- Mechanical chip removal processing
2- Acid treatment
3- Surface treatment
4- Laser processing
5- Pressing

The male can be used to create a mould, for example a deformable one made of silicone, into which the overheated and therefore liquid make-up substance 2 will be cast.

Once solidified, the stick can be extracted from the mould in a conventional manner.

It has therefore been seen how the creation of three-dimensional elements (such as those envisaged by the invention) on a substantially planar surface of any make-up product provides three different visual effects when the product is observed from three different viewpoints.

Various embodiments of the innovation have been disclosed herein, but further embodiments may also be conceived using the same innovative concept, as long as they fall within the scope of the claims.

## Claims

1. A makeup product (1) comprising a makeup substance (2) defining at least one makeup surface, at least part of the makeup surface being a substantially planar surface (4) defined by a plurality of three-dimensional elements (5, 5', 5'') mutually placed side by side, **characterized in that** each three-dimensional element (5) is defined by a first (A), a second (B) and a third face (C), the three faces (A, B, C) being squares arranged as three faces of a regular cube with a vertex (V) in common, on the group of first faces (A) being overall made a first decoration (A1) visible when a user observes the substantially planar surface (4) from a direction substantially normal to the first faces (A), on the group of second faces (B) being overall made a second decoration (B1) visible when a user observes the substantially planar surface (4) from a direction substantially normal to the second faces (B), and on the group of third faces (C) being overall made a third decoration (C1) visible when a user observes the substantially planar surface (4) from a direction substantially normal to the third faces (C).

2. Makeup product (1) according to the preceding claim, in which the vertices (V) of all tridimensional elements lie on a single plane (α).

3. Makeup product according to claim 1, wherein the makeup substance (2) is arranged on a substantially planar support (3).

4. Makeup product (1) according to claim 1, wherein the support (3) is a flat surface housed or defined in a base member (6A) of a container (6) having a lid (6B) hinged to the base member (6A) along an axis (F).

5. A make-up product (1) according to claim 4, wherein the lid (6B) supports a mirror (7) which, at least when the container is open, is facing said substantially planar surface (4), the lid (6B) presenting an opening limiter (10) which blocks its opening when the mirror is angled so that a normal thereof is parallel to the normal of said first faces (A).

6. Make-up product (1) according to claim 5, wherein the first decoration (A1) is made so as to be legible when reflected in the mirror (7).

7. Makeup product (1) according to claim 1, wherein the makeup substance is a compact, a baked, an extruded or a cast product.

8. Makeup product (1) according to claim 1, wherein said decoration (A1, B1, C1) is a drawing or a writing or a combination of a drawing and a writing.

9. Makeup product (1) according to claim 1, wherein said substantially planar surface (4) is made on a lip stick.

## Patentansprüche

1. Schminkprodukt (1), umfassend eine Schminksubstanz (2), die mindestens eine Schminkoberfläche definiert, wobei mindestens ein Teil der Schminkoberfläche eine im Wesentlichen ebene Oberfläche (4) ist, die durch eine Vielzahl von dreidimensionalen Elementen (5, 5', 5") definiert ist, die nebeneinander angeordnet sind, **dadurch gekennzeichnet, dass** jedes dreidimensionale Element (5) durch eine erste (A), eine zweite (B) und eine dritte Fläche (C) definiert ist, wobei die drei Flächen (A, B, C) Quadrate sind, die als drei Flächen eines regelmäßigen Würfels mit einer gemeinsamen Ecke (V) angeordnet sind, wobei auf der Gruppe von ersten Flächen (A) insgesamt ein erstes Dekor (A1) sichtbar gemacht wird, wenn ein Benutzer die im Wesentlichen ebene Oberfläche (4) aus einer Richtung, die im Wesentlichen senkrecht zu den ersten Flächen (A) ist, betrachtet,wobei auf der Gruppe der zweiten Flächen (B) insgesamt ein zweites Dekor (B1) sichtbar gemacht wird, wenn ein Benutzer die im Wesentlichen ebene Oberfläche (4) aus einer Richtung, die im Wesentlichen senkrecht zu den zweiten Flächen (B) ist, betrachtet, und wobei auf der Gruppe von dritten Flächen (C) insgesamt ein drittes Dekor (C1) sichtbar gemacht wird, wenn ein Benutzer die im Wesentlichen ebene Oberfläche (4) aus einer Richtung, die im Wesentlichen senkrecht zu den dritten Flächen (C) ist, betrachtet.

2. Schminkprodukt (1) nach dem vorhergehenden Anspruch, bei dem die Ecken (V) aller dreidimensionalen Elemente auf einer einzigen Ebene (α) liegen.

3. Schminkprodukt nach Anspruch 1, wobei die Schminksubstanz (2) auf einem im Wesentlichen ebenen Träger (3) angeordnet ist.

4. Schminkprodukt (1) nach Anspruch 1, wobei der Träger (3) eine flache Oberfläche ist, die in einem Basisbauteil (6A) eines Behälters (6) untergebracht oder definiert ist, der einen Deckel (6B) aufweist, der entlang einer Achse (F) an dem Basisbauteil (6A) gelenkig angebracht ist.

5. Schminkprodukt (1) nach Anspruch 4, wobei der Deckel (6B) einen Spiegel (7) trägt, der zumindest bei geöffnetem Behälter der im Wesentlichen ebenen Oberfläche (4) zugewandt ist, wobei der Deckel (6B) einen Öffnungsbegrenzer (10) aufweist, der seine Öffnung blockiert, wenn der Spiegel abgewinkelt ist, so dass eine Senkrechte davon parallel zu der Senkrechten der ersten Flächen (A) ist.

6. Schminkprodukt (1) nach Anspruch 5, wobei das erste Dekor (A1) so hergestellt ist, dass es lesbar ist, wenn es in dem Spiegel (7) reflektiert wird.

7. Schminkprodukt (1) nach Anspruch 1, wobei die Schminksubstanz ein kompaktes, ein gehärtetes, ein extrudiertes oder ein gegossenes Produkt ist.

8. Schminkprodukt (1) nach Anspruch 1, wobei das Dekor (A1, B1, C1) eine Zeichnung oder eine Schrift oder eine Kombination aus einer Zeichnung und einer Schrift ist.

9. Schminkprodukt (1) nach Anspruch 1, wobei die im Wesentlichen ebene Oberfläche (4) auf einem Lippenstift hergestellt ist.

## Revendications

1. Produit de maquillage (1) comprenant une substance de maquillage (2) définissant au moins une surface de maquillage, au moins une partie de la surface de maquillage étant une surface sensiblement plane (4) définie par une pluralité d'éléments tridimensionnels (5, 5', 5") mutuellement placés côte à côte, **caractérisé en ce que** chaque élément tridimensionnel (5) est défini par une première (A), une deuxième (B) et une troisième face (C), les trois faces (A, B, C) étant des carrés disposés en trois faces d'un cube régulier avec un sommet (V) en commun, sur le groupe de premières faces (A) étant globalement faite une première décoration (A1) visible lorsqu'un utilisateur observe la surface sensiblement plane (4) à partir d'une direction sensiblement normale aux premières faces (A), sur le groupe de deuxièmes faces (B) étant globalement faite une deuxième décoration (B1) visible lorsqu'un utilisateur observe la surface sensiblement plane (4) à partir d'une direction sensiblement normale aux deuxièmes faces (B), et sur le groupe de troisièmes faces (C) étant globalement faite une troisième décoration (C1) visible lorsqu'un utilisateur observe la surface sensiblement plane (4) à partir d'une direction sensiblement normale aux troisièmes faces (C).

2. Produit de maquillage (1) selon la revendication précédente, dans lequel les sommets (V) de tous les éléments tridimensionnels se trouvent sur un seul plan (α).

3. Produit de maquillage selon la revendication 1, dans lequel la substance de maquillage (2) est agencée sur un support sensiblement plan (3).

4. Produit de maquillage (1) selon la revendication 1, dans lequel le support (3) est une surface plate logée ou définie dans un élément de base (6A) d'un récipient (6) ayant un couvercle (6B) articulé sur l'élément de base (6A) le long d'un axe (F).

5. Produit de maquillage (1) selon la revendication 4, dans lequel le couvercle (6B) supporte un miroir (7) qui, au moins lorsque le récipient est ouvert, fait face à ladite surface sensiblement plane (4), le couvercle (6B) présentant un limiteur d'ouverture (10) qui bloque son ouverture lorsque le miroir est incliné de sorte qu'une normale de celui-ci soit parallèle à la normale desdites premières faces (A).

6. Produit de maquillage (1) selon la revendication 5, dans lequel la première décoration (A1) est faite de manière à être lisible lorsqu'elle est réfléchie dans le miroir (7).

7. Produit de maquillage (1) selon la revendication 1, dans lequel la substance de maquillage est un produit compact, cuit, extrudé ou coulé.

8. Produit de maquillage (1) selon la revendication 1, dans lequel la décoration (A1, B1, C1) est un dessin ou une écriture ou une combinaison d'un dessin et d'une écriture.

9. Produit de maquillage (1) selon la revendication 1, dans lequel la surface sensiblement plane (4) est faite sur un tube de rouge à lèvres.
